# EUROPÄISCHE PATENTSCHRIFT

(11) **EP 2 916 855 B1**
(45) Veröffentlichungstag und Bekanntmachung des Hinweises auf die Patenterteilung: **27.07.2016**
(21) Anmeldenummer: 13789518.1
(22) Anmeldetag: 04.11.2013
(51) Int. Cl.: A61K 38/01, A61K 35/32, A61P 19/10

(54) **KOLLAGENHYDROLYSAT UND DESSEN VERWENDUNG**
COLLAGEN HYDROLYZATE AND USE THEREOF
HYDROLYSAT DE COLLAGÈNE ET SON UTILISATION

(30) Priorität: 06.11.2012 DE 102012110612
(43) Veröffentlichungstag der Anmeldung: 16.09.2015
(73) Patentinhaber: GELITA AG, 69412 Eberbach (DE)
(72) Erfinder: OESSER, Steffen, 24960 Glücksburg (DE); GIESEN-WIESE, Monika, 64739 Höchst (DE); FRECH, Hans-Ulrich, 69469 Weinheim (DE); HAUSMANNS, Stephan, 69126 Heidelberg (DE)
(74) Vertreter: Hoeger, Stellrecht & Partner Patentanwälte mbB
(86) Internationale Anmeldenummer: PCT/EP2013/072894
(87) Internationale Veröffentlichungsnummer: WO 2014/072235

(56) Entgegenhaltungen:
- DE-A1-102010 060 564
- DE-A1-102011 000 997
- OESSER S ET AL: "Stimulation of type II collagen biosynthesis and secretion in bovine chondrocytes cultured with degraded collagen", CELL AND TISSUE RESEARCH, BERLIN, DE, Bd. 311, Nr. 3, 20. März 2003 (2003-03-20) , Seiten 393-399, XP002254608,
- GMEZ-GUILLN M C ET AL: "Functional and bioactive properties of collagen and gelatin from alternative sources: A review", FOOD HYDROCOLLOIDS, ELSEVIER BV, NL, Bd. 25, Nr. 8, 8. Februar 2011 (2011-02-08), Seiten 1813-1827, XP028255533, ISSN: 0268-005X, DOI: 10.1016/J.FOODHYD.2011.02.007 [gefunden am 2011-02-15]

## Beschreibung

Die vorliegende Erfindung betrifft ein neuartiges Kollagenhydrolysat.

Die Erfindung betrifft ferner die Verwendung dieses neuartigen Kollagenhydrolysats als Wirkstoff zur Erhaltung und/oder Verbesserung der Gesundheit der Knochen, insbesondere zur Vorbeugung und/oder Behandlung von Osteoporose. Die Erfindung betrifft des Weiteren ein Nahrungsergänzungsmittel, welches das Kollagenhydrolysat umfasst.

Die Knochen der Wirbeltiere und somit auch die des Menschen verdanken ihre hohe Festigkeit und mechanische Stabilität dem besonderen Aufbau der Knochenmatrix, die von den Osteoblasten (Knochenzellen) synthetisiert wird. Die beiden wesentlichen Komponenten der Knochenmatrix sind zum Einen ein Gerüst aus quervernetztem Kollagen, wobei es sich speziell im Fall der Knochenmatrix um Kollagen vom Typ I handelt. Dessen Quervernetzung erfolgt im Wesentlichen über die Aminosäuren Lysin und Hydroxylysin. Bei der zweiten Komponente handelt es sich um Hydroxylapatit (auch als Apatit-(CaOH) bezeichnet), welches in die Knochenmatrix eingelagert wird (Mineralisation der Knochen). Dieser Aufbau des Knochens ist grob vergleichbar mit demjenigen von Stahlbeton, bei dem sich die Eigenschaften des Stahlgerüsts (entsprechend dem Kollagen) und des Betons (entsprechend dem Hydroxylapatit) ebenfalls unter Ausbildung einer äußerst tragfähigen Struktur ergänzen.

Im Gegensatz zu vielen anderen Gewebetypen weist der Knochen eine relativ hohe Regenerationsfähigkeit auf, d.h. die extrazelluläre Knochenmatrix wird kontinuierlich auf- und wieder abgebaut. Bei einer Störung dieses Gleichgewichts, also einem unzureichenden Aufbau von neuer Knochenmatrix, kommt es zu einem Knochenschwund. Diese Abnahme der Knochendichte wird als Osteoporose bezeichnet und kann verschiedene Ursachen haben. Typischerweise tritt die Osteoporose mit zunehmendem Lebensalter auf (meist ab Beginn des fünften Lebensjahrzehnts), besonders häufig bei Frauen nach der Menopause (postmenopausale Osteoporose).

Es ist seit einiger Zeit bekannt, dass durch die orale Verabreichung von Kollagenhydrolysaten dem Verlust der Knochendichte bei Osteoporose entgegengewirkt werden kann. Kollagenhydrolysate, die durch enzymatische Hydrolyse von tierischem Kollagen gewonnen werden, umfassen jeweils ein Gemisch von Peptiden mit unterschiedlichen Kettenlängen bzw. Molekulargewichten. Das Europäische Patent EP 0 777 491 B1 offenbart z.B. die Verwendung eines Kollagenhydrolysats mit einem mittleren Molekulargewicht von 1.000 bis 40.000 Da, welches durch enzymatische Hydrolyse von Hautkollagen hergestellt ist, zur Behandlung von postmenopausaler Osteoporose. Es wird angenommen, dass dieser vorteilhafte Effekt von Kollagenhydrolysat auf die Gesundheit der Knochen auf einer Stimulation der Biosynthese von Typ-I-Kollagen und anderen Matrixproteinen durch die Osteoblasten beruht, ähnlich wie dies für Chondrozyten bereits *in vitro* nachgewiesen werden konnte (siehe S. Oesser und J. Seiffert (2003), Cell Tissue Research (311) 393-399).

Die DE 10 2011 000 997 A1 offenbart eine Zusammensetzung für Ernährungszwecke mit einer verbesserten Wirksamkeit in Bezug auf den Aufbau der Knochenmatrix, umfassend ca. 1 bis ca. 99 Gew.% Kollagenhydrolysat und ca. 1 bis ca. 99 Gew.% eines oder mehrerer Präbiotika, jeweils bezogen auf die Trockenmasse der Zusammensetzung.

Die DE 10 2010 060 564 A1 offenbart die Verwendung von Kollagenhydrolysat zur Verbesserung der Gesundheit der menschlichen Haut, Haare und/oder Nägel, wobei mindestens 70 Gew.% des Kollagenhydrolysats ein Molekulargewicht von weniger als ca. 3.500 Da aufweisen.

In einem Artikel von M.C. Gómez-Guillén et al. (2011) in Food Hydrocolloids (25) 1813-1827 wird eine Übersicht über die funktionalen und bioaktiven Eigenschaften von Kollagen und Gelatine aus alternativen Quellen, sowie der hieraus hergestellten Hydrolysate, dargestellt.

Verschiedene Kollagenhydrolysate unterscheiden sich hinsichtlich der Molekulargewichtsverteilung der enthaltenen Peptide, deren Aminosäuresequenz und anderer Parameter, in Abhängigkeit vom Ausgangsmaterial und dem Herstellungsverfahren. Der vorliegenden Erfindung liegt die Aufgabe zu Grunde, ein Kollagenhydrolysat vorzuschlagen, welches eine besonders hohe Wirksamkeit in Bezug auf die Erhaltung und/oder Verbesserung der Gesundheit der Knochen aufweist.

Diese Aufgabe wird erfindungsgemäß gelöst durch ein Kollagenhydrolysat, welches durch enzymatische Hydrolyse von Knochengelatine vom Typ B hergestellt ist, wobei das Kollagenhydrolysat aus Peptiden gebildet ist, von denen mindestens 50 Gew.% ein Molekulargewicht von 1.500 bis 13.500 Da aufweisen, und deren mittleres Molekulargewicht im Bereich von 4.500 bis 6.000 Da liegt.

Von den Erfindern durchgeführte Untersuchungen zur Stimulation der Synthese von Matrixproteinen durch Osteoblasten *in vitro,* die weiter unten noch im Detail beschrieben werden, haben überraschenderweise gezeigt, dass ein derartiges Kollagenhydrolysat im Vergleich zu verschiedenen anderen Hydrolysaten eine signifikant höhere stimulierende Wirkung aufweist, insbesondere auf die Synthese von Typ-I-Kollagen. Als besonders vorteilhaft hat sich ein Kollagenhydrolysat erwiesen, bei dem mindestens 70 Gew.% der Peptide ein Molekulargewicht von 1.500 bis 13.500 Da aufweisen.

Besonders interessant ist bei diesem Ergebnis die hohe Wirksamkeit eines Kollagenhydrolysats speziell aus Knochengelatine, zumal aus dem Stand der Technik eher die Verwendung von Hydrolysaten aus Kollagen bzw. Gelatine aus tierischer Haut (vor allem aus Schweineschwarten, aber z.B. auch aus Fischhäuten) bekannt ist. Generell ist Gelatine als denaturierte, lösliche Form des Kollagens ein gut geeignetes Ausgangsmaterial für die enzymatische Hydrolyse. Die im Rahmen der Erfindung als Ausgangsprodukt für das Hydrolysat eingesetzte Knochengelatine ist eine Gelatine vom Typ B, die bevorzugt durch einen alkalischen Aufschluss von Kollagen aus Knochen von Wirbeltieren, insbesondere aus Ossein, hergestellt ist. Bei Ossein handelt es sich um entfettete und demineralisierte Knochen. Günstigerweise wird Ossein aus Rinderknochen eingesetzt.

Der isoelektrische Punkt (IEP) der als Ausgangsmaterial eingesetzten Knochengelatine liegt vorzugsweise unterhalb von 5,5. Demgegenüber weisen Gelatinen vom Typ A, die durch einen sauren Aufschluss des Kollagens hergestellt sind, einen isoelektrischen Punkt oberhalb von 7 auf.

Bei dem erfindungsgemäßen Kollagenhydrolysat weisen die Peptide bevorzugt einen Amidierungsgrad der Glutamin- bzw. Glutaminsäurereste und der Asparagin- bzw. Asparaginsäurereste von insgesamt weniger als 15% auf, insbesondere weniger als 10%. Der Amidierungsgrad wird demnach berechnet durch Division des molaren Anteils der Glutamin- und Asparaginreste durch den molaren Anteil der Glutamin-, Glutaminsäure-, Asparagin- und Asparaginsäurereste in den Peptiden. Der letztgenannte Wert ergibt sich aus der natürlichen Aminosäurezusammensetzung des Kollagens und beträgt typischerweise ca. 1,12 mmol/g. Der molare Anteil der Glutamin- und Asparaginreste kann durch saure Hydrolyse der Amide und Bestimmung des dabei gebildeten Ammoniaks ermittelt werden. Ein niedriger Amidierungsgrad der Peptide kann insbesondere durch den alkalischen Aufschluss der eingesetzten Gelatine (Typ B) erreicht werden.

Die Untersuchungen zur Stimulation der Matrixproteinsynthese durch Osteoblasten haben interessanterweise auch gezeigt, dass das erfindungsgemäße Kollagenhydrolysat mit einem bevorzugten mittleren Molekulargewicht von 4.600 bis 6.000 Da eine höhere Wirksamkeit aufweist als verschiedene niedermolekularere Hydrolysate. Mit dem Molekulargewicht der enthaltenen Peptide korreliert auch die Viskosität des Kollagenhydrolysats. Diesbezüglich ist es bevorzugt, wenn eine 20 Gew.%ige wässrige Lösung des Kollagenhydrolysats bei 25 °C eine Viskosität von mehr als 5 mPa·s aufweist, insbesondere mehr als 6 mPa·s.

Das erfindungsgemäße Kollagenhydrolysat weist vorzugsweise einen Gehalt an Ammonium, Sulfat und Phosphat von jeweils weniger als 300 ppm auf, insbesondere weniger als 100 ppm. Entsprechend niedrige Salzgehalte können bereits bei der Herstellung der für die Hydrolyse eingesetzten Knochengelatine eingehalten werden.

Für die enzymatische Herstellung von Kollagenhydrolysat können verschiedene Proteasen, insbesondere mikrobiellen Ursprungs, eingesetzt werden, wobei deren unterschiedliche Spezifitäten für bestimmte Aminosäuren die molekulare Struktur der resultierenden Peptide und damit auch deren Wirksamkeit direkt beeinflussen. Es hat sich gezeigt, dass ein im Hinblick auf die Stimulation der Osteoblasten besonders wirksames Kollagenhydrolysat bevorzugt durch eine Hydrolyse der Gelatine mit einer neutralen Endoprotease aus *Bacillus subtilis* herstellbar ist.

Gemäß einer bevorzugten Ausführungsform der Erfindung ist das Kollagenhydrolysat durch Einwirkung der Endoprotease bei einer Temperatur von 40 bis 60 °C, insbesondere ca. 50 °C, während eines Zeitraums von 20 bis 40 min, insbesondere ca. 30 min, hergestellt.

Die vorliegende Erfindung betrifft, neben dem vorstehend beschriebenen Kollagenhydrolysat als solchem, insbesondere auch dessen Verwendung als Wirkstoff zur Erhaltung und/oder Verbesserung der Gesundheit der Knochen, insbesondere zur Vorbeugung und/oder Behandlung von Osteoporose. Auf Grund der ausgeprägten stimulierenden Wirkung des erfindungsgemäßen Kollagenhydrolysats auf die Synthese von Matrixproteinen durch die Osteoblasten, die durch Versuche *in vitro* belegt ist, kann durch orale Verabreichung des Kollagenhydrolysats einer Störung des Gleichgewichts zwischen Knochenauf- und -abbau gezielt entgegengewirkt werden.

Ein wesentlicher Aspekt der Erfindung betrifft die Verwendung des Kollagenhydrolysats als Wirkstoff zur Vorbeugung und/oder Behandlung von postmenopausaler Osteoporose. Von dieser Form des Knochenschwundes sind nach Schätzungen etwa 50% der Frauen im Alter von über 50 Jahren betroffen, wobei in den ersten fünf Jahren nach der Menopause bis zu 20% der Knochensubstanz verloren gehen können.

Die vorliegende Erfindung betrifft somit auch ein Verfahren zur Vorbeugung und/oder Behandlung von Osteoporose, insbesondere von postmenopausaler Osteoporose, wobei das Verfahren die orale Verabreichung des erfindungsgemäßen Kollagenhydrolysats an einen Patienten umfasst, insbesondere an eine Patientin im Alter von über 50 Jahren.

Da für Hydrolysate aus tierischen oder pflanzlichen Proteinen eine Zulassung als Arzneimittel nicht erforderlich ist, kann das erfindungsgemäße Kollagenhydrolysat günstigerweise als Nahrungsergänzungsmittel formuliert sein. Das Kollagenhydrolysat kann beispielsweise in Form eines Pulvers, Granulats, einer Lösung oder einer Suspension, oder in Form von Tabletten, Kapseln, Capsetten oder Sachets vorliegen, ggf. in Kombination mit geeigneten Zusatz- oder Hilfsstoffen. Alternativ kann das Kollagenhydrolysat auch direkt einem Lebensmittel zugesetzt werden.

Die tägliche orale Aufnahme liegt vorzugsweise im Bereich von 1 bis 15 g des Kollagenhydrolysats, bevorzugt von 2 bis 10 g, weiter bevorzugt von 2 bis 7 g, und insbesondere im Bereich von 2,5 bis 5 g. Die entsprechende Menge kann günstigerweise als einmalige Tagesdosis formuliert werden.

Eine weitere bevorzugte Ausführungsform der Erfindung betrifft ein Nahrungsergänzungsmittel, welches neben dem erfindungsgemäßen Kollagenhydrolysat ein oder mehrere Präbiotika umfasst. Die Kombination von Kollagenhydrolysat mit einem oder mehreren Präbiotika beruht auf der Überlegung, dass für eine besonders effektive Regeneration der Knochensubstanz nicht nur die Biosynthese von Kollagen und anderen Matrixproteinen, sondern auch die Bildung und Einlagerung von Hydroxylapatit in ausreichender Menge ermöglicht werden muss. Ein limitierender Faktor ist hierbei die Versorgung der Knochenmatrix mit Calcium, wobei nicht die ausreichende Aufnahme von Calcium mit der Nahrung das Problem darstellt (diese ist bei einer ausgewogenen Ernährung in der Regel gewährleistet), sondern die ausreichende Resorption des aufgenommenen Calciums im Darm, die insbesondere aufgrund der Bildung von schwer löslichen Calciumsalzen limitiert ist.

Es hat sich gezeigt, dass die Resorption von Calcium und damit die Mineralisation der Knochen durch die Verabreichung von Präbiotika verbessert werden kann. Bei Präbiotika handelt es sich allgemein um nicht verdaubare Lebensmittelbestandteile, die das Wachstum und/oder die Aktivität bestimmter Mikroorganismen der Darmflora gezielt anregen und dadurch einen positiven Einfluss auf die Gesundheit aufweisen. Der genaue Mechanismus, über den sich dieser Effekt der Präbiotika vorteilhaft auf die Calciumresorption auswirkt, ist zwar noch nicht ganz geklärt, der oben genannte positive Effekt wurde aber z.B. für die Verabreichung von Fruktanen an Kinder im Alter von ca. 12 Jahren belegt (siehe S. Abrams et al. (2005), American Journal of Clinical Nutrition (82) 471-476).

Durch die gemeinsame Verabreichung von Kollagenhydrolysat und Präbiotika kann somit die Bildung der beiden wesentlichen Bestandteile der Knochenmatrix stimuliert und dadurch ein synergetischer Effekt in Bezug auf die Gesundheit der Knochen erzielt werden, insbesondere im Hinblick auf die Vorbeugung und/oder Behandlung von Osteoporose.

Das erfindungsgemäße Nahrungsergänzungsmittel kann prinzipiell jedes beliebige Gewichtsverhältnis von Kollagenhydrolysat und Präbiotikum bzw. Präbiotika umfassen. Um eine ausreichende Versorgung mit beiden Bestandteilen zu gewährleisten, ist es jedoch bevorzugt, wenn der Anteil des Kollagenhydrolysats und des oder der Präbiotika jeweils ca. 20 bis ca. 80 Gew.% beträgt, weiter bevorzugt jeweils ca. 40 bis ca. 60 Gew.%. Das Nahrungsergänzungsmittel kann insbesondere eine Mischung der beiden Bestandteile im Gewichtsverhältnis von etwa 1:1 umfassen.

Das Präbiotikum oder die Präbiotika, die in dem erfindungsgemäßen Nahrungsergänzungsmittel eingesetzt werden, sind bevorzugt ausgewählt aus Oligo- und/oder Polysacchariden. Oligo- und Polysaccharide machen den Großteil der bekannten präbiotisch wirksamen Substanzen aus, wobei sich durch die Verwendung derartiger Substanzen der zusätzliche vorteilhafte Effekt ergibt, dass der Geschmack des Nahrungsergänzungsmittels im Vergleich zu reinem Kollagenhydrolysat deutlich verbessert wird. Obwohl mittels entsprechender Verfahren Kollagenhydrolysate hergestellt werden können, die üblicherweise als geschmacksneutral bezeichnet werden, nehmen viele Verbraucher eine als "leimig" bezeichnete geschmackliche Beeinträchtigung wahr. Überraschenderweise können die negativen geschmacklichen Komponenten von Kollagenhydrolysat durch die Kombination mit präbiotischen Oligo- und/ oder Polysacchariden fast vollständig eliminiert werden.

Das oder die Präbiotika sind bevorzugt ausgewählt aus Inulin, Fruktanen, Galacto-Oligosacchariden (GOS), Fructo-Oligosacchariden (FOS), resistenten Maltodextrinen, Polydextrose und Mischungen hiervon. Diese umfassen sowohl natürlich vorkommende als auch synthetisch hergestellte Saccharide. Bei Inulin handelt es sich um ein Fruktan, welches bis zu 100 Fructose-Einheiten umfasst sowie eine endständige Glucose-Einheit. Fructo-Oligosaccharide und Galacto-Oligosaccharide umfassen ausschließlich Fructose- bzw. Galactose-Einheiten (in der Regel bis zu 10), und bei Polydextrose handelt es sich um ein synthetisches Polysaccharid aus Glucose-, Sorbit- und Zitronensäure-Einheiten.

Weiterhin ist es bevorzugt, wenn das Nahrungsergänzungsmittel als weiteren Bestandteil mindestens ein lösliches Calciumsalz umfasst. Hierdurch wird gleichzeitig eine ausreichende Versorgung des Anwenders mit Calcium, unabhängig von dessen sonstigen Ernährungsgewohnheiten, sichergestellt.

Es kommen prinzipiell alle löslichen Calciumsalze in Frage, die nicht toxisch sind oder sonstige nachteilige Wirkungen haben. Vorzugsweise ist das mindestens eine lösliche Calciumsalz ausgewählt aus Calciumcitrat, Calciumlactat, Calciumgluconat, Calciumlactatgluconat, Calciumlactobionat und Mischungen hiervon.

Das erfindungsgemäße Nahrungsergänzungsmittel kann als Kombinationspräparat auch noch weitere Komponenten enthalten, die einen positiven Effekt auf die Gesundheit der Knochen haben oder die allgemein als Nahrungsergänzung sinnvoll sind. Besonders günstig ist es z.B., wenn das Nahrungsergänzungsmittel ferner ein oder mehrere Vitamine umfasst, die ausgewählt sind aus Vitamin C, Vitamin D, Vitamin D₃, Vitamin E, Vitamin K und deren Metaboliten.

Weiterhin kann das erfindungsgemäße Nahrungsergänzungsmittel auch durch verschiedene Mineralstoffe ergänzt werden, wie insbesondere Fluor-, Kalium- und Magnesiumsalze. Die Aufnahme solcher Mineralien über die Darmwand kann außerdem durch kurzkettige Fettsäuren unterstützt werden.

Eine weitere vorteilhafte Ergänzung stellen Omega-3-Fettsäuren dar, die dazu führen können, den Calcitonin-Gehalt im Knochen zu erhöhen. Außerdem wird ihnen eine anti-inflammatorische Wirkung nachgesagt.

Auch Soja-Isoflavone haben möglicherweise einen positiven Effekt auf die Knochendichte und können in dem erfindungsgemäßen Nahrungsergänzungsmittel eingesetzt werden.

Schließlich sind noch Gewürze bzw. deren Inhaltsstoffe wie z.B. Curcumin und Chili zu nennen, die mit ihrer anti-inflammatorischen und immunmodulatorischen Wirkung zu den vorteilhaften Effekten des erfindungsgemäßen Nahrungsergänzungsmittel beitragen können.

Durch die positive Wirkung des erfindungsgemäßen Kollagenhydrolysats auf die Knochengesundheit ermöglicht dessen Verabreichung als Nahrungsergänzungsmittel unter Umständen einen Verzicht auf oder zumindest eine Dosisreduktion von Arzneimitteln, die sonst zu diesem Zweck eingesetzt werden, insbesondere zur Vorbeugung und/oder Behandlung von postmenopausaler Osteoporose. Beispiele für solche pharmazeutischen Wirkstoffe, deren Anwendung zum Teil nicht unproblematisch ist, sind selektive Östrogenrezeptormodulatoren (SERM), Parathormon und dessen Analogon Teriparatit, weitere Hormone (insbesondere Östrogene und Wachstumshormone), Biphosphonate und monoklonale Antikörper.

Ein weiterer Gegenstand der Erfindung ist ein Verfahren zur Herstellung des erfindungsgemäßen Kollagenhydrolysats, umfassend die Schritte:
- Herstellen einer wässrigen Lösung von Knochengelatine vom Typ B mit einer Konzentration von 5 bis 20 Gew.%, insbesondere ca. 10 Gew.%;
- Zugabe einer neutralen Endoprotease aus *Bacillus subtilis* in einer Menge von 1 bis 4 Gew.%, insbesondere ca. 2 Gew.%, bezogen auf die Menge an Gelatine;
- Einwirken lassen der Endoprotease auf die Gelatine bei einer Temperatur von 40 bis 60 °C, insbesondere ca. 50 °C, und einem pH-Wert von 5,5 bis 6,5, insbesondere ca. 6, während eines Zeitraums von 20 bis 40 min, insbesondere ca. 30 min; und
- thermisches Inaktivieren der Endoprotease.

Besondere Vorteile und bevorzugte Ausführungsformen des erfindungsgemäßen Verfahrens wurden bereits im Zusammenhang mit dem erfindungsgemäßen Kollagenhydrolysat erläutert.

Die nachfolgenden Beispiele dienen unter Bezugnahme auf die Figuren der näheren Erläuterung der Erfindung.

Es zeigen im Einzelnen:
- Figur 1:: ein Gelpermeationschromatogramm mit der Molekulargewichtsverteilung eines erfindungsgemäßen Kollagenhydrolysats und eines Vergleichshydrolysats;
- Figur 2:: ein Diagramm betreffend die Stimulation der Synthese von Typ-I-Kollagen und Osteocalcin durch ein erfindungsgemäßes Kollagenhydrolysat und ein Vergleichshydrolysat; und
- Figur 3:: ein Diagramm betreffend die Stimulation der Synthese verschiedener Matrixproteine und Enzyme durch ein erfindungsgemäßes Kollagenhydrolysat und vier verschiedene Vergleichshydrolysate.

### Herstellung von Kollagenhydrolysaten

Im Folgenden wird die Herstellung eines Kollagenhydrolysats gemäß der vorliegenden Erfindung beschrieben (Beispiel), sowie von vier Kollagenhydrolysaten, die nicht der Erfindung entsprechen (Vergleichsbeispiele 1 bis 4).

Als Ausgangsmaterialien wurden zwei verschiedene Gelatinen eingesetzt, nämlich eine Gelatine vom Typ B aus Rinderknochen (im Folgenden als Knochengelatine bezeichnet) und eine Schweineschwartengelatine vom Typ A. Deren wesentliche Parameter sind in der Tabelle 1 dargestellt.

**Tabelle 1**

| | Knochengelatine | Schweineschwartengelatine |
|---|---|---|
| Bloom-Stärke | 298 g | 299 g |
| Viskosität (6,67 Gew.%, 60 °C) | 5,52 mPa·s | 3,90 mPa·s |
| Leitfähigkeit (1 Gew.%, 30 °C) | 111 µS/cm | 157 µS/cm |
| Feuchte | 10,7% | 9,6% |
| Isoelektrischer Punkt | 5,04 | 9,16 |
| Amidierungsgrad | 6,7% | 31,7% |

### Beispiel

200 g Knochengelatine wurden in 1800 g destilliertem Wasser für 30 min bei Raumtemperatur gequollen. Unter Rühren wurde die vorgequollene Gelatine auf 58±2 °C erwärmt und gelöst. Der pH-Wert wurde mit Natronlauge auf 6,0± 0,2 eingestellt. Danach wurden 2 Gew.% (bezogen auf die Gelatine) einer neutralen Endoprotease aus *Bacillus subtilis* zugegeben. Nach 30 min Hydrolysedauer wurde die Lösung zur Enzyminaktivierung auf 85 °C erwärmt. Zur Gewinnung des gebildeten Kollagenhydrolysats wurde die Lösung getrocknet.

### Vergleichsbeispiel 1

200 g Knochengelatine wurden in 1800 g destilliertem Wasser für 30 min bei Raumtemperatur gequollen. Unter Rühren wurde die vorgequollene Gelatine auf 49±2 °C erwärmt und gelöst, anschließend wurden 1,64 g CaCl₂·2H₂O zugegeben. Der pH-Wert wurde mit Natronlauge auf 6,0±0,2 eingestellt. Danach wurden 0,6 Gew.% (bezogen auf die Gelatine) einer neutralen Endoprotease aus *Bacillus subtilis* zugegeben. Nach 30 min Hydrolysedauer werden 0,2 Gew.% (bezogen auf die Gelatine) einer neutralen Endoprotease aus *Bacillus amyloliquefaciens* zugegeben. Nach insgesamt 180 min Hydrolysedauer wurde die Lösung zur Enzyminaktivierung auf 85°C erwärmt und anschließend getrocknet.

### Vergleichsbeispiel 2

200 g Knochengelatine wurden in 1800 g destilliertem Wasser für 30 min bei Raumtemperatur gequollen. Unter Rühren wurde die vorgequollene Gelatine auf 49±2 °C erwärmt und gelöst, anschließend wurden 0,4 g CaCl₂·2H₂O zugegeben. Der pH-Wert wird mit Natronlauge auf 6,5±0,2 eingestellt. Danach wurden 0,6 Gew.% (bezogen auf die Gelatine) einer neutralen Endoprotease aus *Bacillus subtilis* zugegeben. Nach 60 min Hydrolysedauer wurden 0,2 Gew.% (bezogen auf die Gelatine) einer Exoprotease aus *Aspergillus sojae* zugegeben. Nach insgesamt 7 h Hydrolysedauer wurde die Lösung zur Enzyminaktivierung auf 85°C erwärmt und anschließend getrocknet.

### Vergleichsbeispiel 3

200 g Schweineschwartengelatine wurden in 1800 g destilliertem Wasser für 30 min bei Raumtemperatur gequollen. Unter Rühren wurde die vorgequollene Gelatine auf 57±2 °C erwärmt und gelöst. Der pH-Wert wurde mit Natronlauge auf 6,0±0,2 eingestellt. Danach wurden 0,3 Gew.% (bezogen auf die Gelatine) einer neutralen Endoprotease aus *Bacillus subtilis* zugegeben. Nach 180 min wurde die Lösung zur Enzyminaktivierung auf 85 °C erwärmt und anschließend getrocknet.

### Vergleichsbeispiel 4

200 g Schweineschwartengelatine wurden in 1800 g destilliertem Wasser für 30 min bei Raumtemperatur gequollen. Unter Rühren wurde die vorgequollene Gelatine auf 58±2 °C erwärmt und gelöst. Der pH-Wert wurde mit Natronlauge auf 7,0±0,2 eingestellt. Danach wurden 2 Gew.% (bezogen auf die Gelatine) einer alkalischen Endoprotease aus *Bacillus licheniformis* zugegeben. Nach 180 min wurde die Lösung zur Enzyminaktivierung auf 85°C erwärmt und anschließend getrocknet.

### Bestimmung der Molekulargewichtsverteilung

Die Molekulargewichtsverteilung der Peptide der verschiedenen Kollagenhydrolysate wurde mittels Gelpermeationschromatographie unter Verwendung der folgenden Parameter bestimmt:

| | |
|---|---|
| Stationäre Phase: | TSK 2000 SW XL (Tosoh Bioscience GmbH) |
| Mobile Phase: | 0,4 mol/l Natriumdihydrogenphosphat pH 5,3 |
| Flussrate: | 0,5 ml/min |
| Kalibrierungsstandard: | definierte Kollagen-Typ-I-Fragmente (FILK, Freiberg) |
| Detektion: | UV-Detektor Knauer K-2501 bei 214 nm |

In der Tabelle 2 sind jeweils die Molekulargewichtsverteilung nach vorgegebenen Gewichtsfraktionen, das mittlere Molekulargewicht, die Viskosität und der pH-Wert (jeweils bei 20 Gew.% und 25 °C) sowie der Amidierungsgrad der verschiedenen Kollagenhydrolysate angegeben.

**Tabelle 2**

| Fraktionen | Beispiel | Vgl.-Bsp. 1 | Vgl.-Bsp. 2 | Vgl.-Bsp. 3 | Vgl.-Bsp. 4 |
|---|---|---|---|---|---|
| > 18 kDa | 4,3 | 0,1 | 0 | 0,8 | 0 |
| 13,5-18 kDa | 2,9 | 0,2 | 0,1 | 2,0 | 0 |
| 7,5-13,5 kDa | 21,1 | 5,0 | 0,7 | 20,6 | 0 |
| 3,5-7,5 kDa | 29,8 | 25,4 | 10,6 | 25,8 | 0,9 |
| 1,5-3,5 kDa | 24,5 | 34,6 | 24,3 | 22,8 | 10,9 |
| 0,5-1,5 kDa | 15,1 | 31,0 | 46,4 | 20,5 | 70,9 |
| < 0,5 kDa | 2,4 | 3,7 | 18,0 | 7,4 | 17,4 |
| Mittleres MG | 5.800 Da | 2.900 Da | 1.800 Da | 4.700 Da | 950 Da |
| Viskosität | 6,77 mPa·s | 4,15 mPa·s | 3,22 mPa·s | 5,61 mPa·s | 2,45 mPa·s |
| pH-Wert | 6,2 | 6,1 | 6,4 | 6,0 | 6,5 |
| Amidierungsgrad | 6,8% | N.B. | N.B. | N.B. | 27,6% |

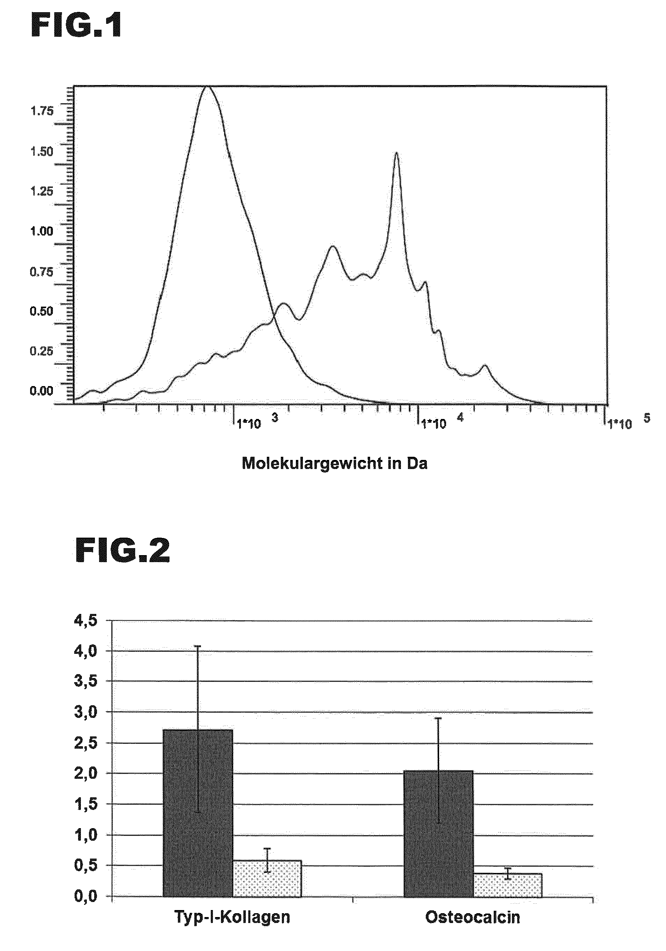

Die Figur 1 zeigt das Gelpermeationschromatogramm mit der Molekulargewichtsverteilung des erfindungsgemäßen Kollagenhydrolysats gemäß dem Beispiel und des Kollagenhydrolysats gemäß dem Vergleichsbeispiel 4. Das Molekulargewicht ist auf der Abszisse mit einer logarithmischen Skala aufgetragen.

### Stimulation des Synthese von Matrixproteinen und Enzymen in vitro

Als Beleg für die besondere Wirksamkeit des erfindungsgemäßen Kollagenhydrolysats wurde dessen stimulierende Wirkung auf die Synthese von Matrixproteinen und Enzymen, die beim Aufbau und der Mineralisation der Matrix eine Rolle spielen, durch Osteoblasten *in vitro* untersucht. Dies erfolgte durch eine Bestimmung der Expression der entsprechenden mRNA mittels Realtime-PCR und eine semiquantitative Auswertung (bezogen auf eine Kontrolle ohne Kollagenhydrolysat).

Die humanen Osteoblasten wurden aus Kniegelenken isoliert, indem Knochenmaterial unter starker Agitation bei 37 °C für 1 h in Hanks-Lösung inkubiert wurde, supplementiert mit 7 mg/ml Hyaluronidase Typ I und III-S und 5 mg/ml Pronase. Der Aufschluss wurde dann bei 37 °C für 3-5 h in Hanks-Lösung, supplementiert mit 16 mg/ml Kollagenase Typ CLS IV, fortgesetzt. Primäre Osteoblasten wurden nach dem enzymatischen Aufschluss kultiviert in HAMs F12-Medium, supplementiert mit 10% fötalem Kälberserum, 20 U/ml Penicillin-Streptomycin, 50 µg/ml Partricin, 0,05 mg/ml Ascorbinsäure und 0,15 mg/ml Glutamin.

Für die eigentliche Untersuchung wurden Monolayer-Zellkulturen der humanen Osteoblasten für einen Zeitraum von 24 h in einem Medium inkubiert, welches mit 0,5 mg/ml des jeweiligen Kollagenhydrolysats (gemäß dem Beispiel oder einem der Vergleichsbeispiele) supplementiert war. Eine Kontrolle wurde jeweils in Medium ohne Hydrolysat inkubiert. Anschließend erfolgte die Bestimmung der jeweiligen mRNA-Expression wie oben beschrieben.

Die Ergebnisse für die Stimulation der Synthese von Typ-I-Kollagen und Osteocalcin sind als Säulendiagramm in der Figuren 2 dargestellt, wobei die linke Säule jeweils für das erfindungsgemäße Beispiel steht und die rechte Säule jeweils für das Vergleichsbeispiel 4. Auf der Ordinate ist die mRNA-Expression relativ zur Kontrolle (=1) aufgetragen (Mittelwert und Standardabweichung aus mindestens 12 unabhängigen Versuchen). Während Typ-I-Kollagen das mit Abstand wichtigste Matrixprotein des Knochenmaterials darstellt, ist Osteocalcin ein an der Differenzierung der Matrix beteiligtes Enzym, dessen verstärkte Expression ebenfalls den Aufbau der Matrix begünstigt.

In beiden Fällen zeigt sich eine deutliche stimulierende Wirkung des erfindungsgemäßen Hydrolysats, nämlich eine Steigerung der Kollagen-mRNA-Expression im Mittel um einen Faktor von ca. 2,7 und der Osteocalcin-mRNA-Expression im Mittel um einen Faktor von ca. 2,0. Interessanterweise führt das Hydrolysat gemäß dem Vergleichbeispiel 4, welches aus einem anderen Ausgangsmaterial hergestellt ist und ein geringeres mittleres Molekulargewicht aufweist, im Unterschied hierzu sogar zu einer Verringerung der Expression der beiden mRNAs, was die hohe Spezifität des erfindungsgemäßen Kollagenhydrolysats zeigt.

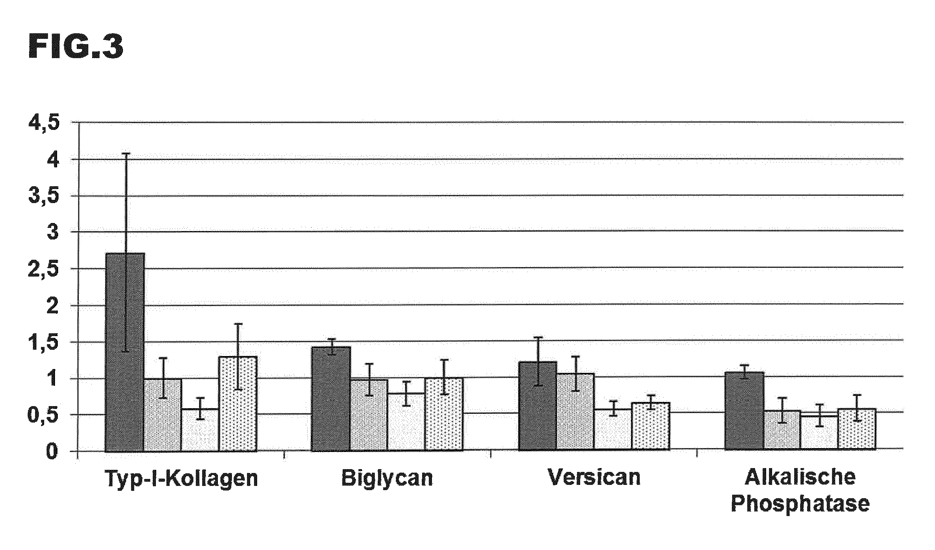

Entsprechende Ergebnisse für die Stimulation der Synthese von Typ-I-Kollagen, Biglykan, Versican und alkalischer Phosphatase sind als Säulendiagramm in der Figur 3 dargestellt, wobei die Säulen von links nach recht jeweils für das erfindungsgemäße Beispiel und die Vergleichsbeispiele 1, 2 und 3 stehen (Mittelwert und Standardabweichung aus mindestens 12 unabhängigen Versuchen). Biglykan und Versican sind Proteoglykane, die eine Rolle bei der strukturellen Organisation der Knochenmatrix spielen. Alkalische Phosphatase ist - neben anderen Funktionen - ähnlich wie Osteocalcin an der Differenzierung der Knochenmatrix beteiligt.

Auch hier zeigt sich für alle vier Proteine, dass das erfindungsgemäße Kollagenhydrolysat eine bessere Wirkung aufweist als alle Vergleichsbeispiele. Von letzteren hat lediglich das Vergleichsbeispiel 3 eine leicht stimulierende Wirkung auf die Synthese von Typ-I-Kollagen (Faktor von ca. 1,3), in allen anderen Fällen ergibt sich keine oder sogar eine negative Wirkung, d.h. eine Reduktion der mRNA-Synthese. Bei Biglykan, Versican und alkalischer Phosphatase ist zwar auch die Wirkung des erfindungsgemäßen Kollagenhydrolysats relativ schwach ausgeprägt, aber sie ist dennoch jeweils positiver als diejenige der anderen Hydrolysate.

Die verbesserte Wirksamkeit des erfindungsgemäßen Kollagenhydrolysats gegenüber verschiedenen anderen Hydrolysaten wird also im Hinblick auf alle untersuchten Matrixproteine und Enzyme bestätigt, deren verstärkte Expression den Aufbau der Knochenmatrix begünstigt.

## Patentansprüche

1. Kollagenhydrolysat, welches durch enzymatische Hydrolyse von Knochengelatine vom Typ B hergestellt ist, wobei das Kollagenhydrolysat aus Peptiden gebildet ist, von denen mindestens 50 Gew.%, insbesondere mindestens 70 Gew.%, ein Molekulargewicht von 1.500 bis 13.500 Da aufweisen, und deren mittleres Molekulargewicht im Bereich von 4.500 bis 6.000 Da liegt.

2. Kollagenhydrolysat nach Anspruch 1, wobei die Knochengelatine durch einen alkalischen Aufschluss von Kollagen aus Knochen von Wirbeltieren, insbesondere aus Ossein, hergestellt ist.

3. Kollagenhydrolysat nach einem der vorangehenden Ansprüche, wobei die Knochengelatine einen isoelektrischen Punkt unterhalb von 5,5 aufweist.

4. Kollagenhydrolysat nach einem der vorangehenden Ansprüche, wobei die Peptide einen Amidierungsgrad der Glutamin- bzw. Glutaminsäurereste und der Asparagin- bzw. Asparaginsäurereste von insgesamt weniger als 15% aufweisen, insbesondere weniger als 10%.

5. Kollagenhydrolysat nach einem der vorangehenden Ansprüche, wobei eine 20 Gew.%ige wässrige Lösung des Kollagenhydrolysats bei 25 °C eine Viskosität von mehr als 5 mPa·s aufweist, insbesondere mehr als 6 mPa·s.

6. Kollagenhydrolysat nach einem der vorangehenden Ansprüche, wobei das Kollagenhydrolysat einen Gehalt an Ammonium, Sulfat und Phosphat von jeweils weniger als 300 ppm aufweist, insbesondere weniger als 100 ppm.

7. Kollagenhydrolysat nach einem der vorangehenden Ansprüche, wobei das Kollagenhydrolysat durch Hydrolyse der Gelatine mit einer neutralen Endoprotease aus *Bacillus subtilis* hergestellt ist.

8. Kollagenhydrolysat nach Anspruch 7, wobei das Kollagenhydrolysat durch Einwirkung der Endoprotease bei einer Temperatur von 40 bis 60 °C, insbesondere ca. 50 °C, während eines Zeitraums von 20 bis 40 min, insbesondere ca. 30 min, hergestellt ist.

9. Kollagenhydrolysat nach einem der vorangehenden Ansprüche zur Verwendung als Wirkstoff zur Erhaltung und/oder Verbesserung der Gesundheit der Knochen, insbesondere zur Vorbeugung und/oder Behandlung von Osteoporose.

10. Kollagenhydrolysat zur Verwendung nach Anspruch 9 als Wirkstoff zur Vorbeugung und/ oder Behandlung von postmenopausaler Osteoporose.

11. Kollagenhydrolysat zur Verwendung nach Anspruch 9 oder 10, wobei das Kollagenhydrolysat als Nahrungsergänzungsmittel formuliert ist, insbesondere in Form eines Pulvers, Granulats, einer Lösung oder einer Suspension, oder in Form von Tabletten, Kapseln, Capsetten oder Sachets.

12. Kollagenhydrolysat zur Verwendung nach Anspruch 11, wobei eine tägliche orale Aufnahme von 1 bis 15 g des Kollagenhydrolysats vorgesehen ist, bevorzugt von 2 bis 10 g, weiter bevorzugt von 2 bis 7 g, insbesondere von 2,5 bis 5 g.

13. Nahrungsergänzungsmittel, umfassend das Kollagenhydrolysat nach einem der vorangehenden Ansprüche und ein oder mehrere Präbiotika, die bevorzugt ausgewählt sind aus Oligo- und/oder Polysacchariden, insbesondere aus Inulin, Fruktanen, Galacto-Oligosacchariden (GOS), Fructo-Oligosacchariden (FOS), resistenten Maltodextrinen, Polydextrose und Mischungen hiervon.

14. Nahrungsergänzungsmittel nach Anspruch 13 ferner umfassend (i) ein oder mehrere lösliche Calciumsalze, die insbesondere ausgewählt sind aus Calciumcitrat, Calciumlactat, Calciumgluconat, Calciumlactatgluconat, Calciumlactobionat und Mischungen hiervon, und/oder (ii) ein oder mehrere Vitamine, die ausgewählt sind aus Vitamin C, Vitamin D, Vitamin D₃, Vitamin E, Vitamin K und deren Metaboliten, und/oder (iii) ein oder mehrere Mineralstoffe, die ausgewählt sind aus Fluor-, Kalium- und Magnesiumsalzen.

15. Verfahren zur Herstellung eines Kollagenhydrolysats nach einem der Ansprüche 1 bis 12, umfassend die Schritte:
- Herstellen einer wässrigen Lösung von Knochengelatine vom Typ B mit einer Konzentration von 5 bis 20 Gew.%, insbesondere ca. 10 Gew.%;
- Zugabe einer neutralen Endoprotease aus *Bacillus subtilis* in einer Menge von 1 bis 4 Gew.%, insbesondere ca. 2 Gew.%, bezogen auf die Menge an Gelatine;
- Einwirken lassen der Endoprotease auf die Gelatine bei einer Temperatur von 40 bis 60 °C, insbesondere ca. 50 °C, und einem pH-Wert von 5,5 bis 6,5, insbesondere ca. 6, während eines Zeitraums von 20 bis 40 min, insbesondere ca. 30 min; und
- thermisches Inaktivieren der Endoprotease.

## Claims

1. Collagen hydrolysate, which is produced by enzymatic hydrolysis of type-B bone gelatin, wherein the collagen hydrolysate is formed from peptides of which at least 50% by weight, in particular at least 70% by weight, have a molecular weight of 1,500 Da to 13,500 Da, and which have a mean molecular weight in the range from 4,500 Da to 6,000 Da.

2. Collagen hydrolysate according to claim 1, wherein the bone gelatin is produced by alkaline breakdown of collagen from bones of vertebrates, particularly from ossein.

3. Collagen hydrolysate according to one of the preceding claims, wherein the bone gelatin has an isoelectric point of below 5.5.

4. Collagen hydrolysate according to one of the preceding claims, wherein the peptides have an overall amidation level of the glutamine groups and/or glutamic acid groups and of the asparagine groups and/or aspartic acid groups of less than 15%, particularly less than 10%.

5. Collagen hydrolysate according to one of the preceding claims, wherein a 20% by weight aqueous solution of the collagen hydrolysate has a viscosity at 25°C of more than 5 mPa·s, in particular more than 6 mPa·s.

6. Collagen hydrolysate according to one of the preceding claims, wherein the collagen hydrolysate has an ammonium, sulphate and phosphate content of less than 300 ppm each, in particular less than 100 ppm.

7. Collagen hydrolysate according to one of the preceding claims, wherein the collagen hydrolysate is produced by hydrolysis of the gelatine with a neutral endoprotease from *Bacillus subtilis.*

8. Collagen hydrolysate according to claim 7, wherein the collagen hydrolysate is produced through the action of the endoprotease at a temperature of 40°C to 60°C, particularly approximately 50°C, over a period of 20 min to 40 min, particularly approximately 30 min.

9. Collagen hydrolysate according to one of the preceding claims for use as an active ingredient to maintain and/or improve the health of the bones, in particular to prevent and/or treat osteoporosis.

10. Collagen hydrolysate for use according to claim 9 as an active ingredient for preventing and/or treating postmenopausal osteoporosis.

11. Collagen hydrolysate for use according to claim 9 or 10, wherein the collagen hydrolysate is formulated as a nutritional supplement, in particular in the form of a powder, a granulate, a solution or a suspension or in the form of tablets, capsules, caplets or sachets.

12. Collagen hydrolysate for use according to claim 11, wherein there is provided a daily oral intake of 1 g to 15 g of the collagen hydrolysate, preferably from 2 g to 10 g, more preferably from 2 g to 7 g, and particularly preferably from 2.5 g to 5 g.

13. Nutritional supplement, comprising the collagen hydrolysate according to one of the preceding claims and one or more prebiotics, which are preferably selected from oligosaccharides and/or polysaccharides, in particular from inulin, fructans, galacto-oligosaccharides (GOS), fructo-oligosaccharides (FOS), resistant maltodextrins, polydextrose and mixtures thereof.

14. Nutritional supplement according to claim 13, further comprising (i) one or more soluble calcium salts which are selected, in particular, from calcium citrate, calcium lactate, calcium gluconate, calcium lactate gluconate, calcium lactobionate and mixtures thereof, and/or (ii) one or more vitamins which are selected from vitamin C, vitamin D, vitamin D₃, vitamin E, vitamin K and the metabolites thereof, and/or (iii) one or more minerals which are selected from fluorine salts, potassium salts and magnesium salts.

15. Method for producing a collagen hydrolysate according to one of the claims 1 to 12, comprising the steps:
- production of an aqueous solution of type-B bone gelatin with a concentration of 5% to 20% by weight, in particular approximately 10% by weight;
- addition of a neutral endoprotease from *Bacillus subtilis* in a quantity of 1% to 4% by weight, particularly approximately 2% by weight, in relation to the quantity of gelatin;
- allowing the endoprotease to act on the gelatin at a temperature of 40°C to 60°C, particularly approximately 50°C, and at a pH value of 5.5 to 6.5, particularly approximately 6, over a period of 20 min to 40 min, particularly approximately 30 min; and
- thermal deactivation of the endoprotease.

## Revendications

1. Hydrolysat de collagène, qui est préparé par hydrolyse enzymatique d'une gélatine osseuse de type B, l'hydrolysat de collagène étant formé de peptides dont au moins 50 % en poids, en particulier au moins 70 % en poids, présentent un poids moléculaire de 1500 à 13 500 Da, et dont le poids moléculaire moyen se situe dans la plage de 4500 à 6000 Da.

2. Hydrolysat de collagène selon la revendication 1, dans lequel la gélatine osseuse est préparée par extraction alcaline du collagène à partir d'os de vertébrés, en particulier d'osséine.

3. Hydrolysat de collagène selon l'une des revendications précédentes, dans lequel la gélatine osseuse présente un point isoélectrique inférieur à 5,5.

4. Hydrolysat de collagène selon l'une des revendications précédentes, dans lequel les peptides présentent un degré d'amidation des résidus de glutamine et d'acide glutamique et des résidus d'asparagine et d'acide asparaginique globalement inférieur à 15 %, en particulier inférieur à 10 %.

5. Hydrolysat de collagène selon l'une des revendications précédentes, dans lequel une solution aqueuse à 20 % de l'hydrolysat de collagène présente à 25 °C une viscosité supérieure à 5 mPa·s, en particulier supérieure à 6 mPa·s.

6. Hydrolysat de collagène selon l'une des revendications précédentes, qui présente une teneur en ammonium, en sulfate, et en phosphate respectivement inférieure à 300 ppm, en particulier inférieure à 100 ppm.

7. Hydrolysat de collagène selon l'une des revendications précédentes, qui est préparé par hydrolyse de la gélatine avec une endoprotéase neutre provenant de *Bacillus subtilis.*

8. Hydrolysat de collagène selon la revendication 7, qui est produit sous l'action de l'endoprotéase à une température de 40 à 60 °C, en particulier d'environ 50 °C, pendant une durée de 20 à 40 min, en particulier d'environ 30 min.

9. Hydrolysat de collagène selon l'une des revendications précédentes pour une utilisation en tant que substance active pour le maintien et/ou l'amélioration de la santé des os, en particulier pour la prévention et/ou le traitement de l'ostéoporose.

10. Hydrolysat de collagène pour une utilisation selon la revendication 9 en tant que substance active pour la prévention et/ou le traitement de l'ostéoporose post-ménopausique.

11. Hydrolysat de collagène pour une utilisation selon la revendication 9 ou 10, qui est formulé en tant que complément alimentaire, en particulier sous la forme d'une poudre, d'un granulé, d'une solution ou d'une suspension, ou sous la forme de comprimés, de gélules, de capsules ou de sachets.

12. Hydrolysat de collagène pour une utilisation selon la revendication 11, prévoyant une prise orale quotidienne de 1 à 15 g d'hydrolysat de collagène, de préférence de 2 à 10 g, encore plus préférentiellement de 2 à 7 g, en particulier de 2,5 à 5 g.

13. Complément alimentaire comprenant l'hydrolysat de collagène selon l'une des revendications précédentes et un ou plusieurs prébiotiques, qui sont choisis de préférence parmi les oligo- et/ou polysaccharides, en particulier parmi l'inuline, les fructanes, les galacto-oligosaccharides (GOS), les fructo-oligosaccharides (FOS), les maltodextrines résistantes, la polydextrose et des mélanges de ceux-ci.

14. Complément alimentaire selon la revendication 13, comprenant en outre (i) un ou plusieurs sels de calcium solubles, qui sont choisis notamment parmi le citrate de calcium, le lactate de calcium, le gluconate de calcium, le gluconate - lactate de calcium, le lactobionate de calcium et des mélanges de ceux-ci, et/ou (ii) une ou plusieurs vitamines, qui sont choisies parmi la vitamine C, la vitamine D, la vitamine D₃, la vitamine E, la vitamine K et leurs métabolites, et/ou (iii) un ou plusieurs minéraux, qui sont choisis parmi les sels de fluor, de potassium et de magnésium.

15. Procédé de production d'un hydrolysat de collagène selon l'une des revendications 1 à 12, comprenant les étapes consistant à :
- préparer une solution aqueuse de gélatine osseuse du type B à une concentration de 5 à 20 % en poids, en particulier à environ 10 % en poids ;
- ajouter une endoprotéase neutre provenant de *Bacillus subtilis* en une quantité de 1 à 4 % en poids, en particulier d'environ 2 % en poids, par rapport à la quantité de gélatine ;
- laisser agir l'endoprotéase sur la gélatine à une température de 40 à 60 °C, en particulier d'environ 50 °C, et à un pH de 5,5 à 6,5, en particulier d'environ 6, pendant une durée de 20 à 40 min, en particulier d'environ 30 min ; et
- inactiver thermiquement l'endoprotéase.
